# EUROPÄISCHE PATENTSCHRIFT

(11) **EP 1 680 171 B1**
(45) Veröffentlichungstag und Bekanntmachung des Hinweises auf die Patenterteilung: **07.03.2018**
(21) Anmeldenummer: 04789811.9
(22) Anmeldetag: 22.10.2004
(51) Int. Cl.: A61M 25/01, A61M 25/00

(54) **VORRICHTUNG, INSBESONDERE SCHLEUSE ODER KATHETER, ZUM ZUMINDEST TEILWEISEN EINFÜHREN IN EINEN KÖRPERGANG**
DEVICE, ESPECIALLY TUBE OR CATHETER, FOR AT LEAST PARTIALLY INTRODUCING INTO A BODY PASSAGE
DISPOSITIF, NOTAMMENT TUBE OU CATHETER, DESTINE A ETRE INTRODUIT AU MOINS PARTIELLEMENT DANS UN CONDUIT DU CORPS

(30) Priorität: 03.11.2003 CH 187303; 21.07.2004 CH 122904
(43) Veröffentlichungstag der Anmeldung: 19.07.2006
(73) Patentinhaber: Von Weymarn-Schärli, Alexander, 4059 Basel (CH)
(72) Erfinder: Von Weymarn-Schärli, Alexander, 4059 Basel (CH)
(74) Vertreter: Herrmann, Johanna
(86) Internationale Anmeldenummer: PCT/CH2004/000635
(87) Internationale Veröffentlichungsnummer: WO 2005/042078

(56) Entgegenhaltungen:
- EP-A- 0 371 486
- WO-A-02/13899
- DE-A- 4 113 265
- US-A- 5 542 938
- US-B1- 6 203 525

## Beschreibung

Die Erfindung bezieht sich auf eine Vorrichtung, insbesondere Schleuse oder Katheter, zum zumindest teilweisen Einführen in einen Körpergang nach dem Oberbegriff des Patentanspruchs 1.

Derartige Vorrichtungen werden beispielsweise in ein Gefäss, wie eine Vene oder eine Arterie, eingeführt. Eine in Form einer sogenannten Schleuse ausgebildete Vorrichtung kann einen Einführer, auch Introducer genannt, sowie ein hämostatisches Ventil aufweisen. Lange Schleusen werden beispielsweise bei der Behandlung von Hirngefässen verwendet. Durch den Gefässzugang in der Leiste ist somit ein langer Weg bis zu den Hirngefässen zurückzulegen. Oft sind die Gefässe bei älteren Menschen auf diesem Weg mit starken Windungen versehen. Dies führt zu einer verminderten Steuerbarkeit und Führbarkeit der betreffenden Schleuse bzw. des betreffenden Katheters oder auch eines Stents.

Während des Einbringens bzw. Einführens sollte die betreffende Vorrichtung eher flexibel sein, um alle Windungen gut passieren zu können. Liegt die Schleusenspitze hingegen einmal am gewünschten Ort, wäre es eher hilfreich, eine Vorrichtung mit grösserer Steifigkeit zu haben, um das Therapiematerial sicher an den Therapieort bringen zu können. Eine weiche Vorrichtung, wie zum Beispiel Schleuse, kann im Aortenbogen jedoch oft zurückfedern, so dass eine präzise Platzierung des Therapiematerials, wie zum Beispiel eines Stents, erschwert ist.

Aus der DE 43 16 330 A1 ist ein Führungsdraht für den medizinischen Bereich bekannt, welcher zum Einführen von Kathetern, Schleusen oder dergleichen in den menschlichen Körper dient. Dieser Führungsdraht ist einstückig aus elastischem Material gefertigt und hat eine erste Stärke, mindestens eine Querschnittsverminderung im Anschluss daran und zumindest einen Abschnitt mit einem Querschnitt entsprechend der ersten Stärke.

Aus der US-A-6 015 402 ist ein Stent mit polygonalem, nämlich sechseckigem, Querschnitt bekannt.

Die DE 35 30 310 C2 offenbart einen Führungsdraht für Katheter für Blutgefässe, welcher einen polygonalen Querschnitt sowie einen Versteifungskern aufweist. Letzterer endet eine bestimmte Strecke vor dem untersucherfernen Ende des Führungsdrahtes.

Ferner ist aus der EP 0 773 037 A2 eine Vorrichtung, Führungsdrahteinheit genannt, bekannt, welche einen Hüllenkörper und einen darin befindlichen Innenkörper umfasst, deren Querschnitte so gestaltet sind, dass ein gegenseitiges Verdrehen oder Verwinden der genannten Körper ausgeschlossen ist. Der Innenkörper ist somit an einer Drehung relativ zum Hüllenkörper aufgrund der nicht kreisförmigen, aufeinander einwirkenden Querschnitte gehindert.

Aus der DE 41 13 265 A1 ist eine Vorrichtung nach dem Oberbegriff des Patentanspruchs 1 bekannt. Diese Vorrichtung zeigt ein Einschubteil, das eine Innenwand, eine Aussenwand und einen zwischen diesen gebildeten, ringförmigen Zwischenraum aufweist. Ferner ist ein Anschluss zum Einleiten und Absaugen eines Fluids in den bzw. aus dem gesamten Zwischenraum vorgesehen. Im Zwischenraum sind Stützkörper vorgesehen, so dass bei einem Evakuieren des Zwischenraums das Einschubteil in einer beliebigen Form, die es vor dem Evakuieren eingenommen hat, versteift wird.

Die EP 0 371 486 A zeigt einen Dilatationskatheter umfassend einen äusseren röhrenförmigen Körper, welcher ein aufblasbares Ballonelement umfasst und einen inneren röhrenförmigen Körper, welcher innerhalb des äusseren röhrenförmigen Körpers angeordnet ist.

Das Dokument US6203525 B1 zeigt einen Katheter, der ein Rohr aufweist, welches eine Schlaufe ausbildet, die in einen Körpergang eingeführt werden kann. Eine derartige Schlaufe erfordert einen Platzbedarf, der ein Vielfaches des Durchmessers des Rohrs beträgt.

Der Erfindung liegt die Aufgabe zugrunde, eine Vorrichtung der eingangs erwähnten Art zu schaffen, welche einfacher und platzsparender ausgebildet ist.

Diese Aufgabe wird erfindungsgemäss durch eine Vorrichtung mit den Merkmalen des Patentanspruchs 1 gelöst. Vorteilhafte Weiterbildungen sind Gegenstand der abhängigen Ansprüche.

Erfindungsgemäss ist die Steuereinrichtung durch Anordnung und Ausbildung von Hüllenkörper und Innenkörper selbst gebildet und weist im ringförmigen Zwischenraum zwischen Hüllen- und Innenkörper keinerlei zusätzliche mechanische Mittel auf. Insofern kann im Gegensatz zu dem letztgenannten Stand der Technik die erfindungsgemäße Vorrichtung einen geringeren Gesamtdurchmesser aufweisen, da die im Stand der Technik genannten Stützkörper, welche beispielsweise wie gegenseitige Verzahnungen oder Riffelungen ausgebildet sind, erfindungsgemäß gerade nicht vorzusehen sind. Vielmehr macht sich die erfindungsgemässe Vorrichtung und deren Steuereinrichtung die Anordnung und Ausbildung von Hüllenkörper und Innenkörper selbst zunutze und kann insofern auf zusätzliche mechanische Mittel im ringförmigen Zwischenraum zwischen Hüllenkörper und Innenkörper vollständig verzichten. Wenn mittels der Steuereinrichtung eine Relativbewegung zwischen den Körpern erschwert oder ausgeschlossen ist, dann dient dies letztlich einer Versteifung der gesamten Vorrichtung, so dass das Therapiematerial sicher an den Therapieort überbracht werden kann, ohne dass die Gefahr beispielsweise eines Herausrutschens der Vorrichtung aus dem Zielgefäss besteht. Die erfindungsgemässe Vorrichtung ist demnach beim Zulassen einer Relativbewegung zwischen Hüllenkörper und Innenkörper ausreichend flexibel und beim Erschweren oder Verhindern einer solchen Relativbewegung zwischen den Körpern hinreichend steif. Die erfindungsgemässe Vorrichtung kann besonders schonend in einen Körpergang eingeführt werden und ist aufgrund ihres Aufbaus anders als die bekannte Vorrichtung nach dem letztgenannten Stand der Technik auch in der Lage, in Körpergänge mit relativ geringem Durchmesser eingeführt zu werden. Im übrigen kann die Verformbarkeit der erfindungsgemässen Vorrichtung im nicht versteiften Normalzustand im Vergleich zu dem letztgenannten Stand der Technik verbessert sein, da es im Zwischenraum zwischen Hüllen- und Innenkörper auch nicht zu unabsichtlichen Verhakungen oder Verzahnungen kommen kann.

Gemäss einer vorteilhaften Weiterbildung ist die Steuereinrichtung auf die Reibung zwischen Hüllenkörper und Innenkörper wirkend ausgebildet, so dass durch eine Reibungserhöhung sich eine Relativbewegung zwischen den genannten Körpern erschweren oder ganz verhindern lässt.

Vorteilhafterweise ist die Steuereinrichtung derart ausgebildet, dass die Reibung zwischen Hüllen- und Innenkörper mechanisch und/oder mittels Druck oder Vakuum, elektrischer Polarisation, Magnetisierung und/oder mittels einer molekularen Veränderung steuerbar ist. Insofern kann die Steuereinrichtung an den jeweiligen Anwendungsfall angepasst sein und beispielsweise bei einer in Form einer langen Schleuse ausgebildeten Vorrichtung anders als im Fall einer in Form einer kurzen Schleuse ausgebildeten Vorrichtung beschaffen sein. Es ist aber auch möglich, die Steuereinrichtung als Kombination mehrerer die Reibung steuernder Mittel auszuführen.

Gemäss einer vorteilhaften Ausführungsform der Erfindung ist das Material von Hüllenkörper und Innenkörper zwar biegbar, jedoch verwindungssteif ausgebildet und weisen Hüllenkörper und Innenkörper jeweils einen vorzugsweise polygonalen Querschnitt derart auf, dass Hüllenkörper und Innenkörper mittels der Steuereinrichtung relativ zueinander so drehbar sind, dass der Innenkörper zumindest teilweise am Hüllenkörper anliegt. Bei dieser Ausführungsform ist die Steuereinrichtung also relativ einfach als mechanisches Mittel geformt. Durch das zumindest teilweise Anliegen des Innenkörpers am Hüllenkörper wird die Reibung zwischen den genannten Körpern erhöht und dadurch eine Relativbewegung zwischen den genannten Körpern zumindest erschwert. Dies trägt zu einer Versteifung der gesamten Vorrichtung bei. Im übrigen kann die genannte Massnahme zur Versteifung der Vorrichtung auch schnell wieder aufgehoben werden, indem Hüllenkörper und Innenkörper wieder in ihre nicht zueinander verdrehte Stellung zurückgedreht werden. In dem letztgenannten Fall ist die Vorrichtung wieder ausreichend flexibel und biegsam.

Gemäss einer Weiterbildung der Erfindung sind Hüllenkörper und Innenkörper jeweils sechseckig ausgebildet, konzentrisch zueinander angeordnet und derart dimensioniert, dass der Innenkörper im zueinander verdrehten Zustand der Körper vorzugsweise mit seinen sämtlichen Ecken an einer Innenwand des Hüllenkörpers anliegt. Bei dieser Ausführungsform ist es daher möglich, die Reibung letztlich an sechs zumindest linienförmigen Stellen zu erhöhen. Dadurch verkanten sich Innenkörper und Hüllenkörper, wodurch die innere Reibung vergrössert wird und sich der Vorrichtungsschaft, wie zum Beispiel der Schleusenschaft, versteift.

Gemäss einer anderen Ausführungsform der Erfindung ist mittels der Steuereinrichtung in/an den Zwischenraum zwischen Hüllen- und Innenkörper ein Druckmedium, vorzugsweise Druckluft, einleitbar oder Vakuum anlegbar. Mit Hilfe einer derartigen Steuereinrichtung lässt sich die Flexibilität bzw. Versteifung der gesamten Vorrichtung besonders schnell einstellen und ändern. Insbesondere Druckluft ist in Häusern, in denen derartige Eingriffe vorgenommen werden, ohne weiteres verfügbar.

Gemäss einer weiteren Ausführungsform der Erfindung sind die Steuereinrichtung und Hüllen- sowie Innenkörper derart ausgebildet, dass magnetische Felder unterschiedlicher Polarität entlang dem Hüllenkörper und entlang dem Innenkörper zum wahlweisen Herbeiführen einer gegenseitigen Anziehung der Körper erzeugbar sind, wobei vorzugsweise Hüllen- und Innenkörper aus einem magnetisierbaren Material, insbesondere einem weichmagnetischen Werkstoff, gefertigt oder mit einer magnetisierbaren Beschichtung versehen sind. Durch die gegenseitige Anziehung bzw. Abstossung der Körper lässt sich die Reibung zwischen diesen ebenfalls effektiv beeinflussen.

Gemäss einer weiteren Ausführungsform sind die magnetischen Felder durch Anlegen einer elektrischen Spannung an Hüllen- und Innenkörper erzeugbar. Damit stehen für die Ausbildung der Steuereinrichtung zum effektiven Beeinflussen der Reibung zwischen Hüllen- und Innenkörper eine Vielzahl von Mitteln zur Verfügung, wodurch die Auswahl der am besten für den jeweiligen Anwendungsfall geeignetsten Steuereinrichtung erleichtert ist.

Ausführungsformen des Erfindungsgegenstandes werden nachfolgend anhand der Zeichnung näher erläutert, wobei alle beschriebenen und/oder bildlich dargestellten Merkmale für sich oder in beliebiger Kombination den Gegenstand der vorliegenden Erfindung unabhängig von ihrer Zusammenfassung in den Ansprüchen oder deren Rückbeziehung bilden. Es zeigen:
- Fig. 1: eine schematische, teilweise Darstellung von Gefässen mit darin teilweise eingeführter Vorrichtung;
- Fig. 2: eine schematische Draufsicht auf eine in Form einer Schleuse ausgebildete Vorrichtung;
- Fig. 3: eine schematische Draufsicht auf ein Teil der Vorrichtung gemäss Fig. 2;
- Fig. 4: einen schematischen Querschnitt durch die Vorrichtung im nicht zueinander verdrehten Zustand;
- Fig. 5: eine schematische Darstellung der Vorrichtung gemäss Fig. 4 im zueinander verdrehten Zustand;
- Fig. 6: eine schematische, teilweise Seitenansicht der Vorrichtung, in welcher die Teile deutlich voneinander beabstandet dargestellt sind;
- Fig. 7: eine schematische Seitenansicht des in Fig. 6 gezeigten Teils der Vorrichtung, in welcher die Teile voneinander beabstandet und gegeneinander verschoben dargestellt sind; und
- Fig. 8: einen schematischen Querschnitt durch eine Vorrichtung gemäss einer anderen Ausführungsform.

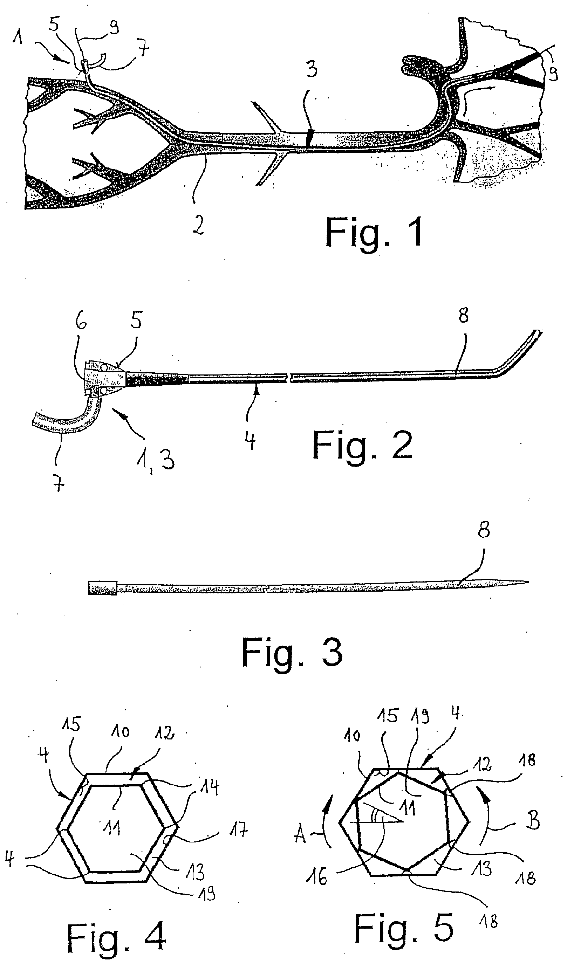

Zunächst wird darauf hingewiesen, dass in den Fig. 1, 2, 4 bis 7 die einen Schnitt symbolisierenden Schraffuren der besseren Übersicht halber weggelassen sind.

In Fig. 1 ist schematisch eine Vorrichtung 1 für den medizinischen Bereich, nämlich beispielhaft eine Schleuse 3, gezeigt, welche teilweise in einen Körpergang 2 eingeführt ist. Die Vorrichtung kann auch als Katheter ausgebildet sein. Der in Fig. 1 gezeigte Körpergang 2 ist ein Teil eines Gefässes, wie zum Beispiel einer Vene oder einer Arterie. Es ist klar, dass der Körpergang 2 in Fig. 1 lediglich stark vereinfacht gezeigt ist.

Die als Schleuse 3 ausgebildete Vorrichtung 1 ermöglicht einen Zugang für eine bestimmte Zeit in den als Gefäss ausgebildeten Körpergang 2, ohne dass dabei zu viel Blut aus dem Gefäss herausfliesst. Der Rückfluss der Flüssigkeit wird gestoppt, obwohl in der Schleuse 3 beispielsweise ein Katheter hin- und hergeschoben wird.

Anders als in Fig. 1 gezeigt, sind die Gefässe im Alter üblicherweise breiter, so dass sie letztlich mehr Windungen machen und mehr Hohlräume aufweisen. Die erfindungsgemässe Vorrichtung soll, wenn sie in das Gefäss hineingeschoben wird, ausreichend flexibel sein, damit sie allen Kurven des Gefässes folgen kann. Wenn die Vorrichtung aber einmal positioniert ist, soll sie möglichst steif sein, damit sie beispielsweise für einen Katheter eine gute Führung bildet.

Wie in Fig. 2 angedeutet, ist die erfindungsgemässe Vorrichtung 1 beispielsweise in Form der Schleuse 3 mit einem länglichen Aussenkörper 4 ausgebildet, an dessen benutzerseitigem Ende 5 sich ein hämostatisches Ventil 6 und ein sogenannter Sideport 7 befinden. Der Sideport 7 dient beispielsweise zum inneren Spülen des länglichen Aussenkörpers 4. In dem länglichen Aussenkörper 4 befindet sich ein sogenannter Einführer, auch Introducer 8 genannt, der das Lumen des länglichen Aussenkörpers 4 nahezu ausfüllt und den Aussenkörper 4 zwecks Einführung der Vorrichtung 1 in den Körpergang 2 versteift.

In dem betreffenden Körpergang 2 liegt gemäss Fig.1 auch ein sogenannter Führungsdraht 9, welcher die Schleuse 3 durchdringt und, wie in Fig. 1 angedeutet, am benutzerseitigen, hinteren Ende 5 und am vorderen Ende der Schleuse 3 aus dieser herausgeführt ist. Der besseren Übersicht halber ist der Führungsdraht 9 in der Darstellung der Schleuse 3 in Fig. 2 weggelassen. Der Führungsdraht 9 dient als Hilfsmittel, entlang welchem die erfindungsgemässe Vorrichtung 1 in das Zielgefäss schiebbar ist.

Der vorgenannte Introducer 8 ermöglicht einen gleitenden Übergang des Durchmesser- bzw. Kaliberunterschiedes vom Durchmesser des Führungsdrahtes 9 auf den Durchmesser des länglichen Aussenkörpers 4.

Gemäss Fig. 4, 5 und 8 hat die erfindungsgemässe Vorrichtung 1 den Aussenkörper 4, welcher einen länglichen äusseren Hüllenkörper 10 und einen von dem Hüllenkörper 10 zumindest abschnittsweise umfangsseitig umgebenen, länglichen Innenkörper 11 aufweist. Die Vorrichtung 1, im gewählten Ausführungsbeispiel die Schleuse 3, ist damit doppelwandig ausgebildet.

Ferner ist eine dem Hüllenkörper 10 und/oder dem Innenkörper 11 zugeordnete Einrichtung 12 vorgesehen, mittels der die Möglichkeit, eine Relativbewegung zwischen Hüllenkörper 10 und Innenkörper 11 zuzulassen oder zumindest zu erschweren, gezielt steuerbar ist.

Erfindungsgemäß ist die Steuereinrichtung 12 durch Anordnung und Ausbildung von Hüllenkörper 10 und Innenkörper 11 selbst gebildet und weist im ringförmigen Zwischenraum 13 zwischen Hüllen- und Innenkörper 10, 11 keinerlei zusätzliche mechanische Mittel auf.

Die Steuereinrichtung 12 ist auf die Reibung zwischen Hüllenkörper 10 und Innenkörper 11 wirkend ausgebildet, wobei der Zwischenraum 13 zwischen Hüllenkörper 10 und Innenkörper 11 in der Darstellung gemäss den Fig. 4, 5 und 8 der besseren Deutlichkeit halber stark vergrössert dargestellt ist.
Gemäss den in den Fig. 4 bis 8 gezeigten Ausführungsformen ist die Steuereinrichtung 12 derart ausgebildet, dass die Reibung zwischen Hüllen- und Innenkörper 10, 11 mechanisch und/oder mittels Druck oder Vakuum, elektrischer Polarisation, Magnetisierung und/oder mittels einer molekularen Veränderung steuerbar ist. Daraus folgt, dass die Steuereinrichtung 12 auf einer der genannten Massnahmen oder auf einer Kombination mehrerer Massnahmen beruht.
Gemäss einer ersten Ausführungsform der Erfindung, welche in den Fig. 4 und 5 gezeigt ist, ist das Material von Hüllenkörper 10 und Innenkörper 11 zwar biegbar, jedoch verwindungssteif ausgebildet. Hüllenkörper 10 und Innenkörper 11 weisen jeweils einen polygonalen Querschnitt derart auf, dass Hüllenkörper 10 und Innenkörper 11 mittels der Steuereinrichtung 12 relativ zueinander so drehbar sind, dass der Innenkörper 11 zumindest teilweise am Hüllenkörper 10 anliegt. Dabei kann die Drehung beispielsweise des Innenkörpers 11 in Richtung des Pfeils A oder in Richtung des Pfeils B relativ zum Hüllenkörper 10 erfolgen (siehe Fig. 5).
Wie in den Fig. 4 und 5 gezeigt, sind Hüllenkörper 10 und Innenkörper 11 jeweils sechseckig ausgebildet und konzentrisch zueinander angeordnet. Sie sind ferner derart dimensioniert, dass der Innenkörper 11 im zueinander verdrehten Zustand der Körper 10, 11 mit seinen sämtlichen sechs Ecken 14 an einer Innenwand 15 des Hüllenkörpers 10 anliegt. In Fig. 4 sind Hüllen- und Innenkörper 10, 11 im nicht zueinander verdrehten Zustand, in Fig. 5 im zueinander verdrehten Zustand gezeigt.
Die Drehung des Innenkörpers 11 kann beispielsweise über einen Winkel 16 von knapp 30° erfolgen. Es ist klar, dass der Winkel kleiner ausgebildet ist, falls die Stärke des Zwischenraums 13 abnimmt.

Wie in Fig. 5 lediglich im rechten Bereich angedeutet, sind an der Innenwand 15 des Hüllenkörpers 10 mehrere längs verlaufende, vorzugsweise gleichmässig über den Innenumfang 17 der Innenwand 15 voneinander beabstandete Rippen 18 vorgesehen. Die Rippen 18 sind dabei derart angeordnet und ausgebildet, dass die Ecken 14 des Innenkörpers 11 bei einer Drehung des Innenkörpers in Richtung des Pfeils A oder des Pfeils B an den Rippen anliegen.

Gemäss einer anderen, nicht näher gezeigten Ausführungsform ist mittels der Steuereinrichtung 12 in den Zwischenraum 13 zwischen Hüllen- und Innenkörper 10, 11 ein Druckmedium, vorzugsweise Druckluft, einleitbar oder an den Zwischenraum 13 ein Vakuum anlegbar. In diesem Fall ist wenigstens einer der genannten Körper aus einem dehnbaren Material gefertigt. Die Erhöhung der Reibung zwischen Hüllen- und Innenkörper 10, 11 erfolgt in dem letztgenannten Fall durch flächiges Anliegen des Innenkörpers 11 an der Innenwand 15 des Hüllenkörpers 10. Dabei kann die Steuereinrichtung beispielsweise so ausgebildet sein, dass der Zwischenraum 13 zwischen Hüllen- und Innenkörper 10, 11 evakuiert oder dass der Innenraum 19 des Innenkörpers 11 mit dem Druckmedium beaufschlagt wird, sich in radialer Richtung dehnt und flächig an der Innenwand 15 des Hüllenkörpers 10 anliegt.

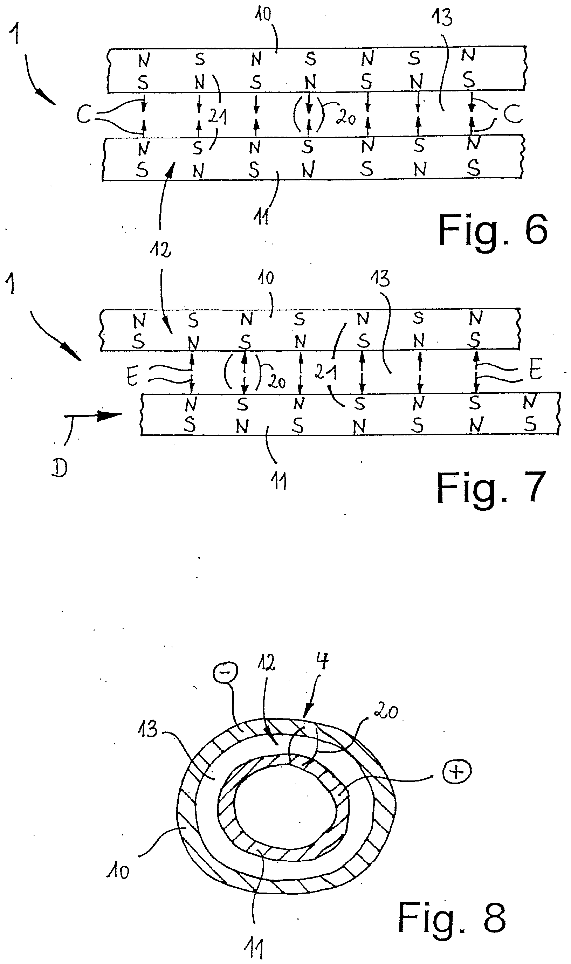

Gemäss weiterer Ausführungsformen der Erfindung, welche einerseits in den Fig. 6 und 7 sowie andererseits in Fig. 8 schematisch dargestellt sind, sind die Steuereinrichtung 12 und Hüllen- sowie Innenkörper 10, 11 derart ausgebildet, dass magnetische Felder 20 unterschiedlicher Polarität 21 entlang dem Hüllenkörper 10 und entlang dem Innenkörper 11 zum wahlweisen Herbeiführen einer gegenseitigen Anziehung der Körper 10, 11 erzeugbar sind.

Gemäss der in den Fig. 6 und 7 dargestellten Ausführungsform der Erfindung sind die magnetischen Felder sowohl innerhalb jedes der Körper 10, 11 als auch von einem Körper zum anderen dauermagnetisch erzeugbar, wobei, wie im einzelnen in einer Seitenansicht auf die Vorrichtung 1 gemäss Fig. 6 gezeigt, die einzelnen Körper über den Zwischenraum 13 voneinander getrennt sind, jeder der Körper 10, 11 entlang seiner Länge und in radialer Richtung abwechselnd umgekehrt polarisiert ist. Dadurch ergibt sich bei einer axialen Anordnung der Körper gemäss Fig. 6 eine gegenseitige Anziehung der Körper 10, 11, wie dies durch die zueinander gerichteten Pfeile C in Fig. 6 verdeutlicht ist.

Wird nun, wie in Fig. 7 angedeutet, beispielsweise der Innenkörper 11 in Richtung des Pfeils D relativ zu dem Hüllenkörper 10 in axialer Richtung verschoben, so stossen sich die Körper 10, 11 voneinander ab, da gleiche Polaritäten sich in den Körpern einander gegenüber liegen. Dies ist durch die voneinander weg gerichteten Pfeile E in Fig. 7 verdeutlicht. Es ist klar, dass der in Fig. 7 gezeigte Effekt des sich Abstossens der Körper ausgehend von der Anordnung gemäss Fig. 6 auch dadurch erreicht werden kann, dass der Hüllenkörper 10 relativ zum Innenkörper 11 in axialer Richtung verschoben wird.

Die unterschiedliche Polarität 21 von Hüllenkörper 10 und Innenkörper 11 ist beispielsweise in den Fig. 6 und 7 durch die Buchstaben "N" und "S" bzw. in Fig. 8 durch die Bezeichnung "+" oder "-" angedeutet, wobei "N" für Nordpol und "S" für Südpol des Magnetfeldes und "+" und "-" für eine positive bzw. negative elektrische Ladung stehen.

Die in den Fig. 6 und 7 durch die Kurzbezeichnungen "N" und "S" angegebenen Polaritäten verlaufen, wie vorstehend bereits angedeutet, einmal innerhalb jedes Körpers, dann aber auch von einem Körper zum anderen.

Hüllen- und Innenkörper 10, 11 sind gemäss einer bevorzugten Ausführungsform der Erfindung aus einem magnetisierbaren Material, insbesondere einem weichmagnetischen Werkstoff, gefertigt oder mit einer magnetisierbaren Beschichtung (nicht näher gezeigt) versehen.

Die Körper 10, 11 sind in der Ausführungsform der Fig. 6 und 7 in Form von Dauermagneten gebildet.

Bei der in Fig. 8 gezeigten Ausführungsform der Erfindung haben sowohl der Hüllenkörper 10 als auch der Innenkörper 11 einen etwa kreisförmigen Querschnitt. Auch bei dieser Ausführungsform ist klar, dass der zwischen dem Hüllenkörper 10 und dem Innenkörper 11 dargestellte Zwischenraum 13 stark vergrössert ist und dass in der Praxis der Aussendurchmesser des Innenkörpers 11 nur geringfügig kleiner als der Innendurchmesser des Hüllenkörpers 10 ist. Sofern erwünscht, kann sich in dem ringförmigen Zwischenraum 13 auch eine Gleitflüssigkeit befinden. Es ist aber auch möglich, eine solche Gleitflüssigkeit wegzulassen.

Bei der in Fig. 8 gezeigten Ausführungsform sind die magnetischen Felder 20 durch Anlegen einer elektrischen Spannung an Hüllen- und Innenkörper 10, 1 1 erzeugbar.

In dem Fall, dass mittels der Steuereinrichtung in die Vorrichtung ein Druckmedium bzw. an die Vorrichtung ein Vakuum einleitbar bzw. anlegbar ist, kann das Druckmedium beispielsweise in den Innenraum 19 des Innenkörpers anlegbar sein. Es ist auch möglich, den Innenkörper doppelwandig auszubilden und dadurch beispielsweise durch Aufblasen eine radiale Dehnung des Innenkörpers herbeizuführen.

Im Falle magnetischer Felder können diese beispielsweise nicht allein über das Material von Hüllenkörper und Innenkörper, sondern auch über eine magnetisierbare Flüssigkeit auf- und abgebaut werden.

Letztlich dienen sämtliche Ausführungsformen dazu, die Reibung zwischen Hüllen-und Innenkörper in der gewünschten Weise auf einfache und platzsparende Art zu beeinflussen.

Damit ist eine Vorrichtung, insbesondere eine medizinische Vorrichtung, geschaffen, welche einfacher und platzsparender handhabbar bzw. ausgebildet, vor allem auf der einen Seite ausreichend flexibel, auf der anderen Seite aber auch hinreichend steif ist, je nachdem, welcher Zustand in der Praxis gewünscht wird.

## Patentansprüche

1. Vorrichtung, insbesondere Schleuse (3) oder Katheter, zum zumindest teilweisen Einführen in einen Körpergang (2), welche einen länglichen, äusseren Hüllenkörper (10), einen von dem Hüllenkörper (10) zumindest abschnittsweise umfangsseitig umgebenen, länglichen Innenkörper (11) und eine Steuereinrichtung (12) aufweist, mittels der die Möglichkeit, eine Relativbewegung zwischen Hüllenkörper (10) und Innenkörper (11) zwecks Herbeiführung einer Versteifung oder einer Flexibilität der gesamten Vorrichtung zuzulassen oder zumindest zu erschweren, gezielt steuerbar ist, **dadurch gekennzeichnet, dass** die Steuereinrichtung (12) durch Anordnung und Ausbildung von Hüllenkörper (10) und Innenkörper (11) selbst gebildet ist und dass entweder das Material von Hüllenkörper (10) und Innenkörper (11) zwar biegbar, jedoch verwindungssteif ausgebildet ist und Hüllenkörper (10) und Innenkörper (11) jeweils einen polygonalen Querschnitt derart aufweisen, dass Hüllenkörper (10) und Innenkörper (11) mittels der Steuereinrichtung (12) relativ zueinander so drehbar sind, dass der Innenkörper (11) zumindest teilweise am Hüllenkörper (10) anliegt, oder dass die Steuereinrichtung (12) und Hüllen- sowie Innenkörper (10,11) derart ausgebildet sind, dass magnetische Felder (20) unterschiedlicher Polarität (21) entlang dem Hüllenkörper (10) und entlang dem Innenkörper (11) zum wahlweisen Herbeiführen einer gegenseitigen Anziehung der Körper (10,11) erzeugbar sind.

2. Vorrichtung nach Anspruch 1, **dadurch gekennzeichnet, dass** der Hüllenkörper (10) und der Innenkörper (11) jeweils sechseckig ausgebildet, konzentrisch zueinander angeordnet und derart dimensioniert sind, dass der Innenkörper (11) im zueinander verdrehten Zustand der Körper (10,11) vorzugsweise mit seinen sämtlichen Ecken (14) an einer Innenwand (15) des Hüllenkörpers (10) anliegt.

3. Vorrichtung nach Anspruch 1 oder 2, **dadurch gekennzeichnet, dass** mittels der Steuereinrichtung (12) in/an einen ringförmigen Zwischenraum (13) zwischen Hüllen-und Innenkörper (10, 11) ein Druckmedium, vorzugsweise Druckluft, einleitbar oder ein Vakuum anlegbar ist.

4. Vorrichtung nach einem der vorhergehenden Ansprüche, **dadurch gekennzeichnet, dass** Hüllen-und Innenkörper (10,11) aus einem magnetisierbaren Material, insbesondere einem weichmagnetischen Werkstoff, gefertigt oder mit einer magnetisierbaren Beschichtung versehen sind.

5. Vorrichtung nach einem der vorhergehenden Ansprüche, **dadurch gekennzeichnet, dass** die magnetischen Felder (20) durch Anlegen einer elektrischen Spannung an Hüllen-und Innenkörper (10,11) erzeugbar sind.

## Claims

1. Device, in particular tube (3) or catheter for at least partial introduction into a body passage (2), comprising a lengthy outer envelope body (10), a lengthy inner body (11) at least sectionally enclosed by the envelope body (10) and a control device (12), by which the possibility to enable or to impede a relative movement of the envelope body (10) and the inner body (11) to increase the stiffness or the flexibility of the entire device is selectively controllable, **characterized in that** the control device (12) is formed by arrangement and configuration of the envelope body (10) and the inner body (11) themselves and that the material of the envelope body (10) and the inner body (11) is flexible but torsionally resistant and the envelope body (10) and the inner body (11) each are disposed with a polygonal cross-section, such that the envelope body (10) and the inner body (11) are rotatable relative to each other by the control device (12) such that the inner body (11) is connected at least partially to the envelope body (10) or the control device (12) and the envelope body (10) and the inner body (11) are configured such that magnetic fields (20) of different polarity (21) can be generated along the envelope body (10) and along the inner body (11) to selectively obtain a mutual attraction of the bodies (10, 11).

2. The device according to claim 1, **characterized in that** the envelope body (10) and the inner body (11) are each of hexagonal configuration, are arranged concentrically with respect to each other and are dimensioned such, that the inner body (11) is connected in the twisted state of the bodies (10, 11) advantageously with all edges (15) to an inner wall (15) of the envelope body (10).

3. The device according to claim 1 or 2, **characterized in that** a pressure medium, preferably pressurized air, can be introduced or a vacuum can be applied by means of the control device (12) in/to an annular intermediate space (13) between the envelope and the inner body (11, 12).

4. The device according to one of the preceding claims, **characterized in that** the envelope body (10) and the inner body (11) are made from a magnetizable material, in particular a soft magnetic material or are provided with a magnetizable coating.

5. The device according to one of the preceding claims, **characterized in that** the magnetic fields (20) can be generated by applying an electrical voltage to the envelope body (10) and the inner body (11).

## Revendications

1. Dispositif, en particulier un tuyau (3) ou cathéter, pour une introduction au moins partielle dans un conduit corporel (2), qui comprend un corps enveloppant (10) externe oblong, un corps interne (11) oblong entouré au moins partiellement par le corps enveloppant (10) et un dispositif de commande (12), par laquelle la possibilité est réglable sélectivement de permettre ou d'aggraver une rigidité ou une flexibilité du dispositif entier par un mouvement relatif entre le corps enveloppant (10) et le corps interne (11), **caractérisé en ce que** le dispositif de commande (12) est formé par l'arrangement et la configuration du corps enveloppant (10) et du corps interne (11) même, et **en ce que** le matériau du corps enveloppant (10) et du corps interne (11) est plutôt flexible, mais rigide envers la torsion et le corps enveloppant (10) et le corps interne (11) ayant une section polygonale pour que le corps enveloppant (10) et le corps interne (11) peuvent rôtir relativement entre eux par voie du dispositif de commande (12) **en ce que** le corps interne (11) est connecté au moins partiellement au corps enveloppant (10) ou le dispositif de commande (12) et le corps enveloppant (10) et le corps interne (11) sont configurés pour générer des champs magnétiques (20) d'une polarité (21) opposée le long du corps enveloppant (10) et du corps interne (11) pour obtenir une attraction mutuelle optionnelle entre les corps (10, 11).

2. Dispositif selon la revendication 1, **caractérisé en ce que** le corps enveloppant (10) et le corps interne (11) sont de la configuration hexagonale et sont arrangés concentriquement et sont dimensionnés d'une façon que le corps interne (11) est avantageusement connecté par tous ses coins au mur de refend du corps enveloppant (10) dans l'état tordu des corps (10, 11).

3. Dispositif selon une des revendications 1 ou 2, **caractérisé en ce qu'**un médium de pression, préférablement l'air comprimé peut être admis ou un vide peut être fait par le dispositif de commande (12) dans un espace intermédiaire annulaire (13) entre le corps enveloppant (10) et le corps interne (11).

4. Dispositif selon une des revendications précédentes **caractérisé en ce que** le corps enveloppant (10) et le corps interne (11) sont fabriqués d'un matériau magnétisable, en particulier d'un matériau de fabrication magnétique souple ou sont revêtis d'une couche magnétisable.

5. Dispositif selon une des revendications précédentes **caractérisé en ce que** les champs magnétiques (20) peuvent être générés par mise sous tension électrique le corps enveloppant (10) et le corps interne (11).
